# FASCICULE DE BREVET EUROPEEN

(11) **EP 3 638 652 B1**
(45) Date de publication et mention de la délivrance du brevet: **23.08.2023**
(21) Numéro de dépôt: 18739897.9
(22) Date de dépôt: 12.06.2018
(51) Int. Cl.: C07D 265/16, C08G 73/06, C09J 179/00

(54) **BENZOXAZINE SULFUREE UTILISABLE POUR LA SYNTHESE D'UNE POLYBENZOXAZINE**
GESCHWEFELTES BENZOXAZIN ZUR VERWENDUNG BEI DER SYNTHESE EINES POLYBENZOXAZINS
SULPHURISED BENZOXAZINE FOR USE IN THE SYNTHESIS OF A POLYBENZOXAZINE

(30) Priorité: 14.06.2017 FR 1755350
(43) Date de publication de la demande: 22.04.2020
(73) Titulaire: Compagnie Générale des Etablissements Michelin, 63000 Clermont-Ferrand (FR)
(72) Inventeur: FEDURCO, Milan, 63040 Clermont-Ferrand Cedex 9 (FR); RIBEZZO, Marco, 63040 Clermont-Ferrand Cedex 9 (FR)
(74) Mandataire: Louret, Sylvain
(86) Numéro de dépôt international: PCT/FR2018/051367
(87) Numéro de publication internationale: WO 2018/229415

(56) Documents cités:
- WO-A1-2014/063968
- POORTEMAN MARC ET AL: "Thermal curing ofpara-phenylenediamine benzoxazine for barrier coating applications on 1050 aluminum alloys", PROGRESS IN ORGANIC COATINGS, ELSEVIER BV, NL, vol. 97, 8 avril 2016 (2016-04-08), pages 99-109, XP029557542, ISSN: 0300-9440, DOI: 10.1016/J.PORGCOAT.2016.03.026

## Description

### 1. DOMAINE DE L'INVENTION

La présente invention est relative aux monomères utilisables pour la synthèse de résines thermodurcissables, destinées notamment à des systèmes adhésifs permettant en particulier le collage de métal à du caoutchouc.

Elle est plus particulièrement relative aux composés benzoxazine aptes à la synthèse de polybenzoxazines utilisables notamment comme couches adhésives dans des composites métal / caoutchouc destinés à la fabrication d'articles en caoutchouc tels que des bandages, pneumatiques ou non pneumatiques, pour véhicules.

### 2. ETAT DE LA TECHNIQUE

Les composites métal / caoutchouc, en particulier pour bandages pour véhicules automobiles, sont bien connus. Ils sont le plus souvent constitués d'une matrice en caoutchouc insaturé généralement diénique, réticulable au soufre, comportant des éléments de renforcement (ou « renforts ») métalliques tels que des fils, films, rubans ou câbles en acier au carbone.

Soumis à des contraintes très importantes lors du roulage des bandages, notamment à des compressions, flexions ou variations de courbure répétées, ces composites doivent de manière connue satisfaire à un grand nombre de critères techniques, parfois contradictoires, tels qu'uniformité, flexibilité, endurance en flexion et en compression, résistance à la traction, à l'usure et à la corrosion, et maintenir ces performances à un niveau très élevé aussi longtemps que possible.

On comprend aisément que l'interphase adhésive entre caoutchouc et renforts joue un rôle prépondérant dans la pérennité de ces performances. Le procédé traditionnel pour relier les compositions de caoutchouc à de l'acier au carbone consiste à revêtir la surface de l'acier avec du laiton (alliage cuivre-zinc), la liaison entre l'acier et la matrice de caoutchouc étant assurée par sulfuration du laiton lors de la vulcanisation ou cuisson du caoutchouc. Pour améliorer l'adhésion, on utilise en outre généralement, dans ces compositions de caoutchouc, des sels organiques ou des complexes de métal tels que des sels de cobalt, en tant qu'additifs promoteurs d'adhésion.

Or, on sait que l'adhésion entre l'acier au carbone et la matrice de caoutchouc est susceptible de s'affaiblir au cours du temps, du fait de l'évolution progressive des sulfures formés sous l'effet des différentes sollicitations rencontrées, notamment mécaniques et/ou thermiques, le processus de dégradation ci-dessus pouvant être accéléré en présence d'humidité. D'autre part, l'utilisation de sels de cobalt rend les compositions de caoutchouc plus sensibles à l'oxydation et au vieillissement, et en augmente significativement le coût, sans compter qu'il est souhaitable de supprimer à terme l'emploi de tels sels de cobalt dans les compositions de caoutchouc, en raison de l'évolution récente de la réglementation européenne sur ce type de sels métalliques.

Pour toutes les raisons exposées ci-dessus, les fabricants de composites métal / caoutchouc, en particulier les manufacturiers de bandages pour véhicules automobiles, sont à la recherche de solutions adhésives nouvelles pour faire coller les renforts métalliques aux compositions de caoutchouc, tout en palliant, au moins en partie, les inconvénients précités.

C'est ainsi que les demandes WO 2014/063963, WO 2014/063968, WO 2014/173838, WO 2014/173839 récemment publiées, déposées par les Demanderesses, ont décrit des polymères nouveaux à unités urée, uréthane ou thio-urée, ainsi que leurs monomères de départ, qui répondent aux objectifs ci-dessus. Utilisés notamment comme primaire d'adhésion sur métal dans des composites métal / caoutchouc, ces polymères permettent très avantageusement de coller le métal aux matrices de caoutchouc en utilisant ensuite de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc lorsque ces dernières contiennent par exemple des élastomères insaturés fonctionnalisés appropriés tels que des élastomères époxydés. Ainsi peuvent être supprimés notamment les sels de cobalt (ou autres sels métalliques) dans les compositions de caoutchouc destinées à être reliées à des renforts métalliques laitonnés.

Poursuivant leurs recherches, les Demanderesses ont trouvé un composé benzoxazine nouveau, utilisable comme monomère pour la synthèse d'une polybenzoxazine, du type thermodurcissable, qui à température ambiante présente les mêmes performances adhésives, vis-à-vis du métal et du caoutchouc, que les polymères précités mais qui présente une fois thermodurcie (réticulée) une stabilité thermique et chimique encore améliorée et dont la microstructure spécifique, en outre, permet très avantageusement d'ajuster la flexibilité de la molécule selon les applications particulières visées.

### 3. BREVE DESCRIPTION DE L'INVENTION

La présente invention concerne une benzoxazine sulfurée répondant à la formule (A) : 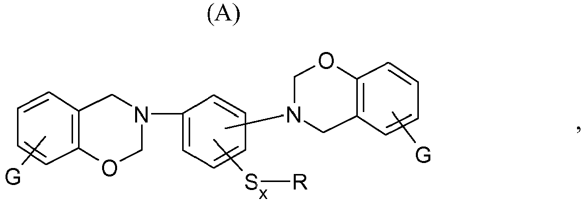 dans laquelle :
- chaque noyau benzénique des deux cycles oxazine est porteur d'au moins un radical noté « G » ;
- les deux cycles oxazine sont reliés entre eux par un groupe aromatique central dont le cycle benzénique est porteur de un, deux, trois ou quatre groupements de formule -Sₓ-R dans laquelle « x » est un entier de 1 à 8 et R représente l'hydrogène ou un groupe hydrocarboné comportant 1 à 10 atomes de carbone et optionnellement un hétéroatome choisi parmi O, S, N et P ;
- les au moins deux radicaux G, identiques ou différents, sont choisis dans le groupe constitué par :
   - les halogènes ;
   - les groupements -OR₁, -SR₁, -NR₂R₃ ; R₁, R₂ et R₃, identiques ou différents, représentant un alkyle ayant 1 à 4 atomes de carbone ; et
   - les groupements hydrocarbonés aliphatiques comportant 1 à 8 atomes de carbone, ou cycloaliphatiques comportant 3 à 8 atomes de carbone, ou aromatiques comportant 6 à 12 atomes de carbone, ces groupements hydrocarbonés, éthyléniquement saturés ou insaturés, comportant en outre optionnellement au moins un hétéroatome choisi parmi O, S, N et P.

Grâce à cette benzoxazine spécifique, il est possible de préparer des polymères benzoxazine ou « polybenzoxazines » qui ont la capacité remarquable, à haute température, d'ouvrir leurs cycles oxazine et de conduire ainsi à une structure de résine polyphénolique thermodurcissable. Ceci leur confère, comparativement aux autres polymères connus décrits en introduction du présent mémoire, une meilleure stabilité thermique. Sa microstructure spécifique permet enfin, très avantageusement, d'ajuster la flexibilité des polybenzoxazines selon les applications particulières visées.

L'invention concerne également l'utilisation d'un composé conforme à l'invention pour la synthèse d'une polybenzoxazine, ainsi que toute polybenzoxazine issue d'au moins un composé benzoxazine selon l'invention.

L'invention concerne également tout procédé de synthèse d'une polybenzoxazine par polycondensation d'un composé selon l'invention, notamment avec, à titre de second monomère, un composé diol ou thiol aromatique.

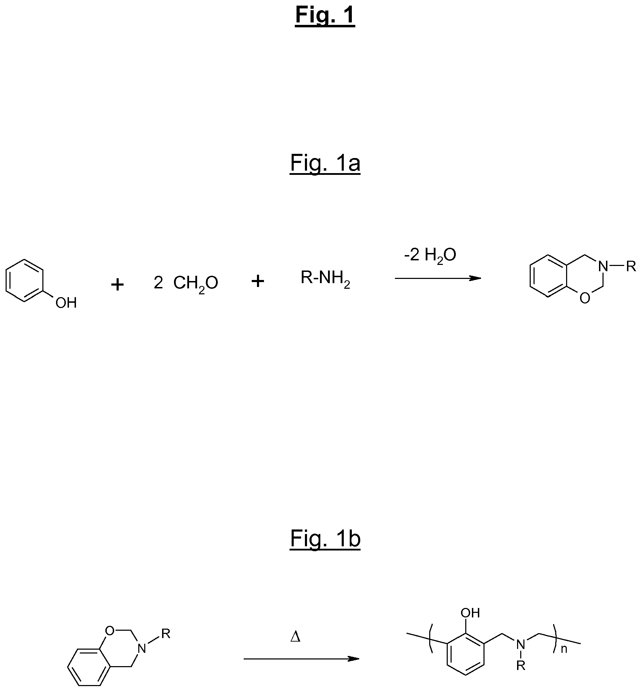

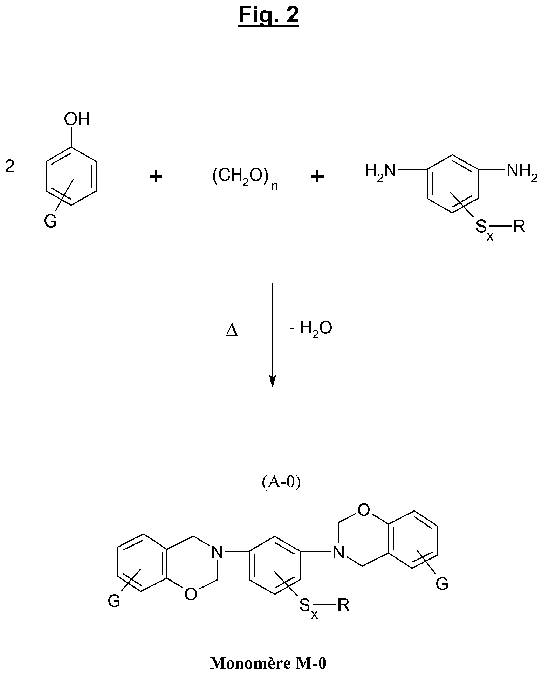

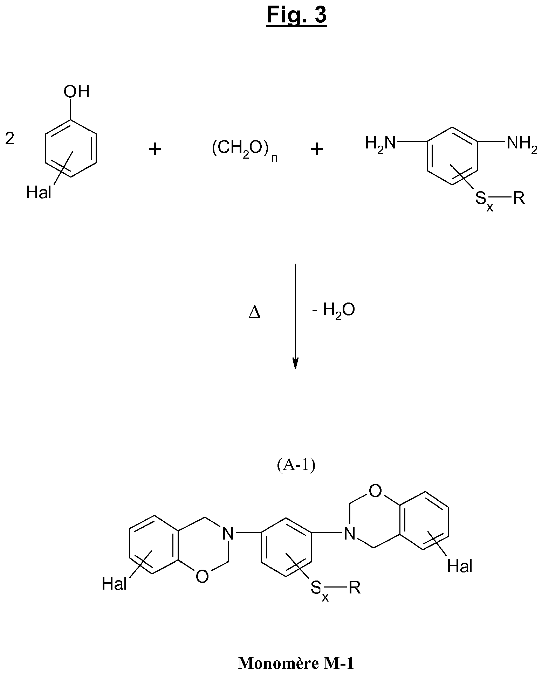

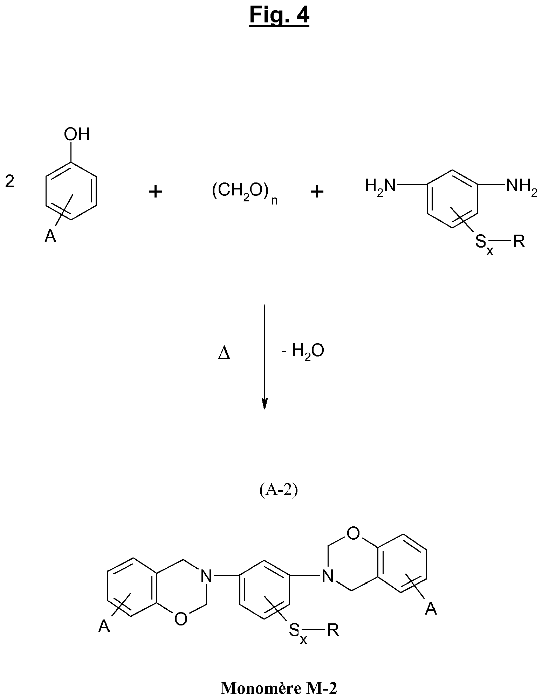

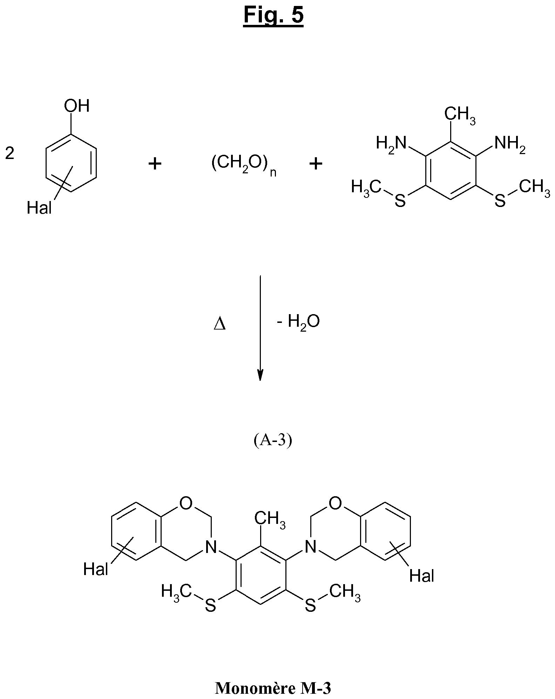

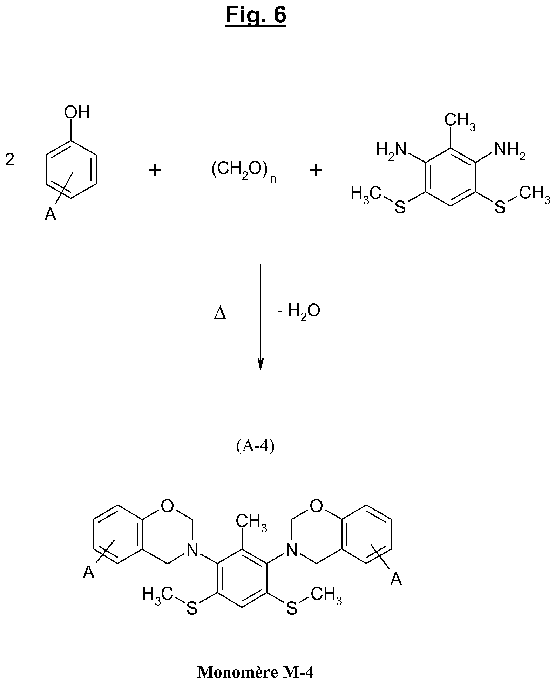

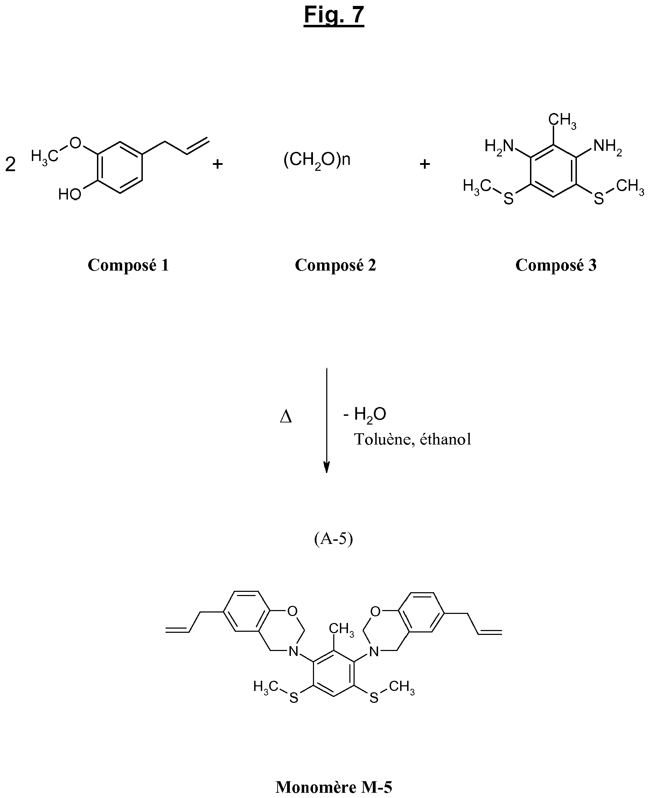

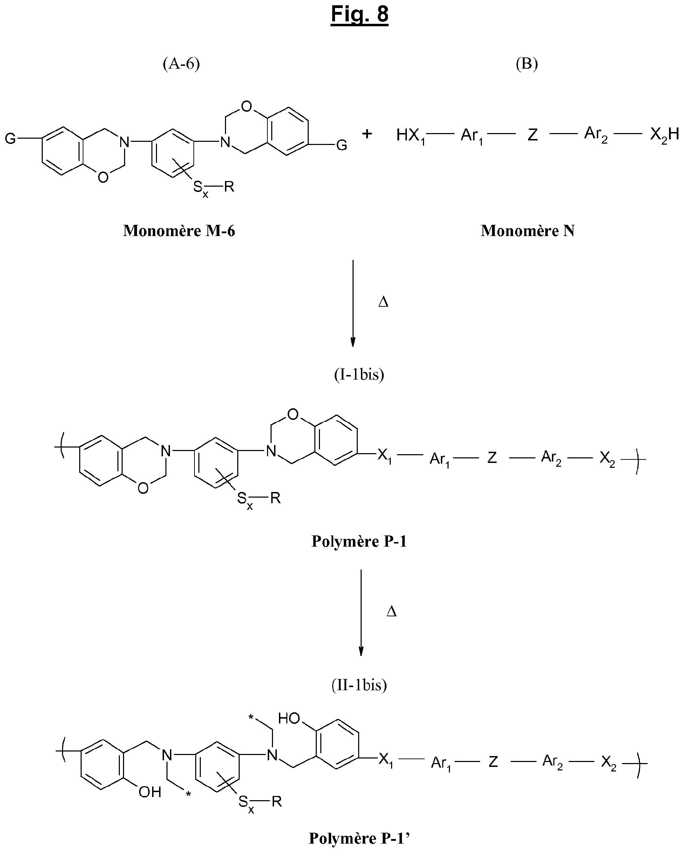

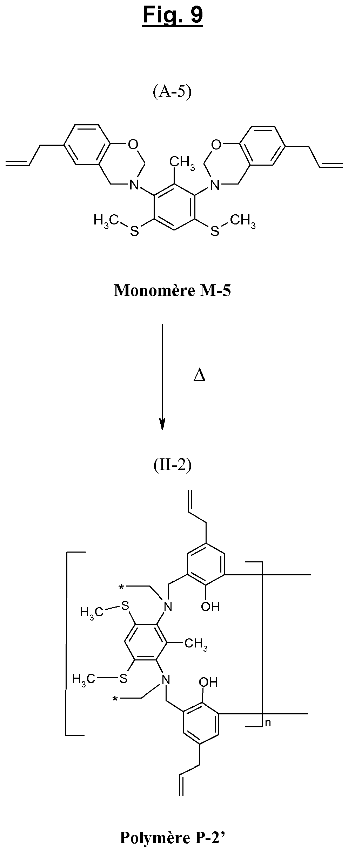

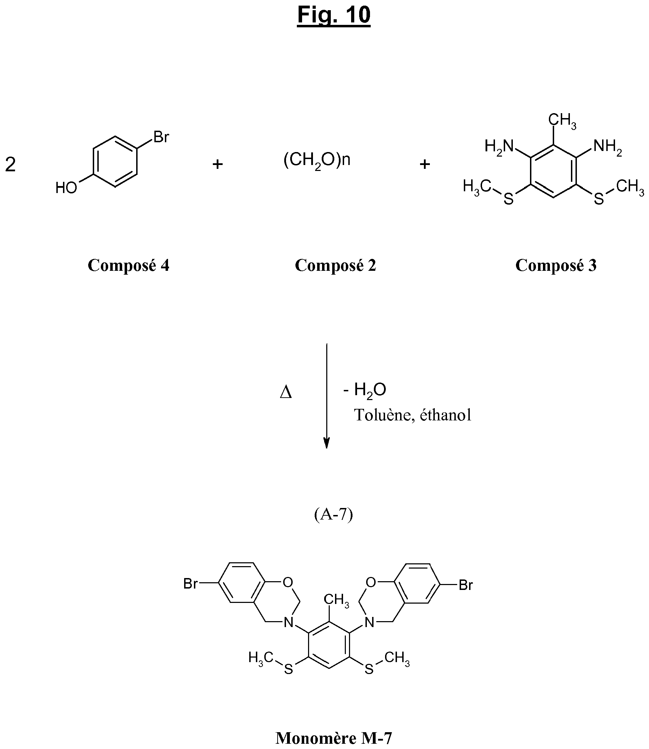

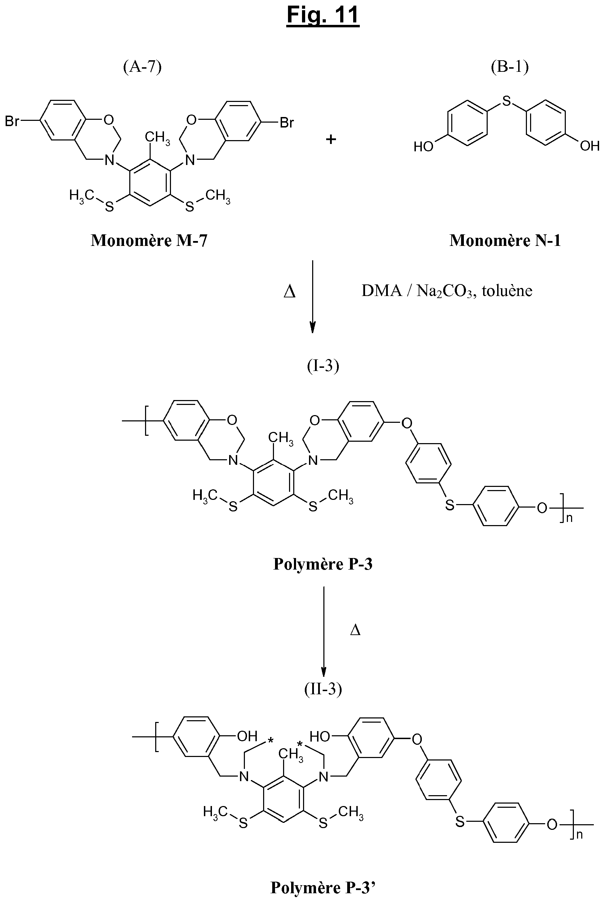

L'invention ainsi que ses avantages seront aisément compris à la lumière de la description détaillée et des exemples de réalisation qui suivent, ainsi que des figures 1 à 11 qui représentent ou schématisent :
- le principe général de synthèse d'un composé benzoxazine à partir de trois composés, phénol, formaldéhyde et amine (R résidu de l'amine) (Fig. 1a) ;
- le mécanisme d'ouverture, par apport thermique, du cycle oxazine (« *ring-opening »)* d'un tel composé benzoxazine (Fig. 1b) ;
- un schéma de synthèse général, à partir d'un phénol spécifique (le symbole « G » sera décrit en détail ultérieurement), de paraformaldéhyde et d'une diamine aromatique sulfurée spécifique, d'une benzoxazine conforme à l'invention de formule (A-0) (Monomère noté « M-0 ») utilisable pour la synthèse d'une polybenzoxazine sulfurée (Fig. 2) ;
- un schéma de synthèse possible, à partir d'un phénol halogéné (le symbole « Hal » représentant un halogène), de p-formaldéhyde et de la diamine aromatique sulfurée spécifique précédente, d'une benzoxazine halogénée particulière conforme à l'invention de formule (A-1) (Monomère noté M-1), utilisable pour la synthèse d'une polybenzoxazine (Fig. 3) ;
- un autre schéma de synthèse possible, à partir d'un autre phénol spécifique (le symbole « A » sera décrit en détail ultérieurement), de p-formaldéhyde et de la diamine aromatique sulfurée spécifique précédente, d'une autre benzoxazine de formule (A-2) (Monomère noté M-2) conforme à l'invention (Fig. 4) ;
- un autre schéma de synthèse possible, à partir d'un phénol halogéné, de p-formaldéhyde et d'un exemple particulier de diamine aromatique sulfurée, d'un autre exemple de benzoxazine halogénée particulière conforme à l'invention, de formule (A-3) (Monomère noté M-3) (Fig. 5) ;
- un autre schéma de synthèse possible, à partir d'un autre phénol spécifique, de p-formaldéhyde et de l'exemple particulier de diamine aromatique sulfurée précédente, d'un autre exemple de benzoxazine conforme à l'invention, de formule (A-4) (Fig. 6) ;
- un autre schéma de synthèse possible, à partir d'un exemple particulier de phénol (Composé 1 ; méthoxyphénol porteur d'une insaturation éthylénique), de p-formaldéhyde (Composé 2) et de l'exemple particulier (Composé 3) de diamine aromatique disulfurée précédente, d'un autre exemple de benzoxazine conforme à l'invention, de formule (A-5) (Monomère noté M-5) (Fig. 7) ;
- un schéma de synthèse général d'un exemple de polybenzoxazine sulfurée (Polymère noté P-1), à partir de la benzoxazine halogénée conforme à l'invention de formule (A-6) (Monomère M-6) précédente et d'un autre monomère de formule générique (B) (Monomère noté « N ») du type diol ou thiol aromatique ; ainsi que cet exemple de polybenzoxazine sulfurée (Polymère noté ici P-1') s une fois ses cycles oxazine ouverts après traitement thermique du Polymère P-1 (Fig. 8) ;
- un schéma de synthèse d'une autre polybenzoxazine (Polymère P-2'), avec ses cycles oxazine ouverts, obtenu par homopolymérisation de la benzoxazine halogénée particulière de formule (A-5) (Monomère M-5) précédente (Fig. 9) ;
- un exemple de synthèse, à partir de phénol bromé (composé 4), p-formaldéhyde (composé 2) et d'une diamine aromatique disulfurée spécifique (composé 3), d'une benzoxazine bromée particulière selon l'invention, de formule (A-7) (Monomère noté M-7) utilisable pour la synthèse de polybenzoxazines (Polymère P-3 et P-3' de la figure 11) (Fig. 10) ;
- enfin, un exemple de synthèse d'une polybenzoxazine sulfurée (Polymère P-3), à partir de la benzoxazine halogénée particulière selon l'invention de formule (A-7) (Monomère M-7) précédente et d'un autre monomère particulier de formule (B-1) (Monomère N-1) du type diol aromatique soufré (porteur d'une fonction thioéther), ainsi que la structure de ce polymère une fois ses cycles oxazine ouverts (Polymère noté P-3') (Fig. 11).

### 4. DESCRIPTION DETAILLEE DE L'INVENTION

On rappellera tout d'abord que les benzoxazines sont des composés de formule générale :

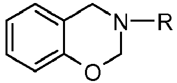

La figure 1a annexée rappelle le principe général de synthèse d'une benzoxazine, ici à partir (réaction de condensation) d'une molécule de phénol, de deux molécules de formaldéhyde et d'une amine (R désignant le résidu de l'amine), avec élimination de deux molécules d'eau.

La figure 1b rappelle quant à elle le mécanisme d'ouverture (« *ring-opening* ») du cycle oxazine d'un tel composé lors d'un apport thermique (représenté par le symbole Δ).

De nombreux composés ou monomères benzoxazines peuvent être ainsi synthétisés en utilisant divers phénols et amines selon leurs types de substituants. Ces groupes substituants peuvent fournir ensuite des sites polymérisables et permettre la synthèse de divers polymères benzoxazine (ou polybenzoxazines).

Benzoxazines et polybenzoxazines qui en sont issues sont des produits aujourd'hui bien connus de l'homme du métier ; pour ne citer que quelques exemples de publication, on peut mentionner les articles « Polybenzoxazines - New high performance thermosetting resins : synthesis and properties » ; N.N. Ghosh et al., Prog. Polym. Sci. 32 (2007), 1344-1391, ou « Recent Advancement on Polybenzoxazine - A newly Developed High Performance Thermoset », Y. Yaggi et al., J. Polym. Sci. Part A: Polym. Chem. : Vol. 47 (2009), 5565-5576, ainsi que par exemple les brevets ou demandes de brevet US 5 543 516, WO 2013/148408.

Comme expliqué en détail dans les documents ci-dessus, les polybenzoxazines ont la capacité remarquable, à haute température (par exemple, typiquement supérieure à 150°C voire à 200°C selon leur microstructure particulière), d'ouvrir leurs cycles oxazine et de conduire ainsi à des structures de résines polyphénoliques thermodurcissables.

La benzoxazine spécifique objet de l'invention (dénommée « Monomère M » dans la présente demande) est du type benzoxazine sulfurée ; elle répond à la formule générique (A) qui suit : 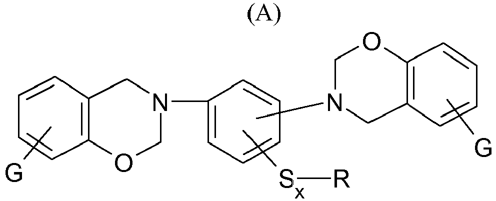 dans laquelle chaque noyau benzénique des deux cycles oxazine est porteur d'au moins un (c'est-à-dire un ou plusieurs) radical G ; la benzoxazine elle-même porte donc au moins deux radicaux G.

Les (au moins) deux radicaux G, identiques ou différents, sont choisis dans le groupe constitué par :
- les halogènes ;
- les groupements -OR₁, -SR₁, -NR₂R₃ ; R₁, R₂ et R₃, identiques ou différents, représentant un alkyle ayant 1 à 4 atomes de carbone ; et
- les groupements hydrocarbonés aliphatiques comportant 1 à 8 atomes de carbone, ou cycloaliphatiques comportant 3 à 8 atomes de carbone, ou aromatiques comportant 6 à 12 atomes de carbone, ces groupements hydrocarbonés, éthyléniquement saturés ou insaturés, comportant en outre optionnellement au moins un hétéroatome choisi parmi O, S, N et P.

Dans cette formule (A), les deux cycles oxazine sont reliés entre eux par un groupe aromatique central dont le cycle ou noyau benzénique (dit aussi cycle ou noyau benzénique central) est porteur de un, deux, trois ou quatre groupements de formule -Sₓ-R dans laquelle « x » est un entier de 1 à 8 et R représente l'hydrogène ou un groupe hydrocarboné comportant 1 à 10 atomes de carbone et optionnellement un hétéroatome choisi parmi O (oxygène), S (soufre), N (azote) et P (phosphore).

De même, on peut noter dans cette formule (A) que les deux atomes d'azote des cycles oxazine sont, l'un par rapport à l'autre, dans une position quelconque (c'est-à-dire ortho, méta ou para) sur le noyau benzénique central. Toutefois, de préférence, ces deux atomes d'azote sont en position méta l'un par rapport à l'autre ; en d'autres termes, la benzoxazine (Monomère dans ce cas noté M-0) de l'invention répond alors préférentiellement à la formule générique (A-0) qui suit :

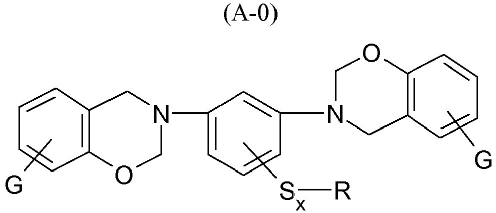

La figure 2 annexée donne le schéma de synthèse général de cette benzoxazine de formule (A-0), sous apport thermique et avec élimination d'eau, à partir d'un phénol spécifique porteur d'au moins un (c'est-à-dire un ou plusieurs) radical G, de paraformaldéhyde et enfin d'une diamine aromatique sulfurée spécifique de formule : 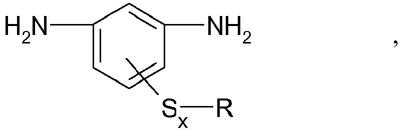 formule dans laquelle, bien entendu, le noyau benzénique est porteur de un, deux, trois ou quatre groupements de formule -Sₓ-R tels que définis précédemment, et pourrait être porteur d'éventuels autres substituants (à titre d'exemple un groupe méthyle ou éthyle).

Préférentiellement, dans cette benzoxazine de formule (A) ou (A-0), le noyau benzénique central est porteur de deux groupements de formule -Sₓ-R, ces deux groupements étant plus préférentiellement en position méta l'un par rapport à l'autre sur ce noyau benzénique central. Selon un autre mode de réalisation préférentiel, « x » est compris dans un domaine de 1 à 4, plus préférentiellement égal à 1 ou 2. R est préférentiellement un alkyle ayant plus préférentiellement 1 à 5 atomes de carbone, encore plus préférentiellement un méthyle ou un éthyle, en particulier un méthyle.

La polybenzoxazine (Polymère « P »), dérivant de la benzoxazine de l'invention de formule (A) précédemment décrite, a donc quant à elle pour caractéristique essentielle de comporter des unités structurelles récurrentes comportant au moins un motif répondant aux formule (I) (avant ouverture des cycles oxazine) ou formule (II) (après ouverture des cycles) ci-dessous :

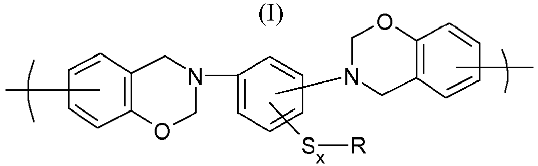

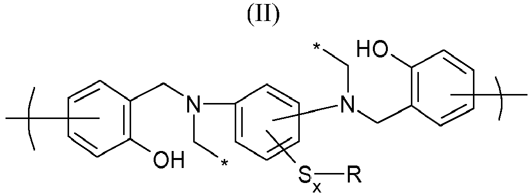

Par polymère doit être entendu dans la présente demande tout homopolymère ou copolymère, notamment copolymère à blocs, avec des unités structurelles récurrentes comportant au moins un motif de formule (1) ou (II) ci-dessus ; le polymère peut bien entendu comporter à la fois des motifs de formule (I) et des motifs de formule (II).

Dans la formule (II) ci-dessus, l'homme du métier comprendra immédiatement que les deux symboles « * » (identiques ou différents) représentent un rattachement quelconque du motif à un atome de carbone ou à un hétéroatome (choisi de préférence parmi O, S, N et P), rattachement ou liaison résultant de l'ouverture des cycles oxazine lors d'un apport thermique (Δ) suffisant.

En outre, dans les formules (I) et (II) ci-dessus, comme pour le monomère de formule (A), un ou plusieurs atomes d'hydrogène d'au moins un ou de chaque noyau benzénique des deux cycles oxazine, ainsi que ceux du cycle benzénique central, pourraient optionnellement être également substitués par divers substituants (à titre d'exemple un groupe méthyle ou éthyle), notamment par des groupes fonctionnels (à titre d'exemple un groupe vinyle) susceptibles de favoriser l'adhésion du polymère au métal et/ou au caoutchouc.

De même, comme pour le monomère de formule (A) précédente, on peut noter dans ces formules (1) et (II) que les deux atomes d'azote des cycles oxazine sont, l'un par rapport à l'autre, dans une position quelconque (c'est-à-dire ortho, méta ou para) sur le noyau benzénique central qui les sépare.

Toutefois, de préférence, ces deux atomes d'azote sont en position méta l'un par rapport à l'autre sur le noyau benzénique central ; en d'autres termes, la polybenzoxazine issue du composé benzoxazine de l'invention comporte alors au moins des unités structurelles récurrentes comportant (au moins) un motif répondant aux formule (1-bis) (avant ouverture des cycles oxazine) ou formule (11-bis) (après ouverture des cycles) ci-après :

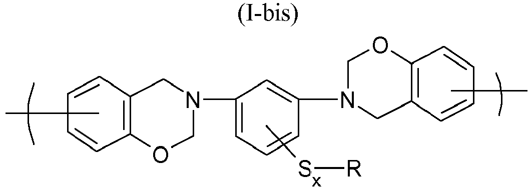

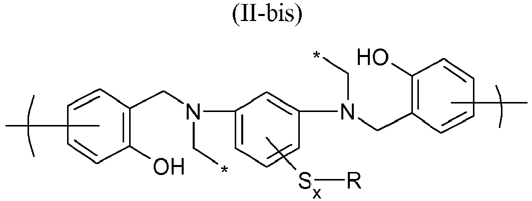

Dans les formules (A) ou (A-0) précédente du composé de l'invention, de préférence, chaque noyau benzénique des deux cycles oxazine est porteur d'un seul radical G ou au maximum de deux, plus préférentiellement d'un et d'un seul radical G.

Ce dernier (un seul radical G) est encore plus préférentiellement situé en position para de l'oxygène du cycle oxazine ; la benzoxazine de l'invention répond donc dans ce cas à la formule qui suit (notée (A-6) sur la figure 8 annexée) :

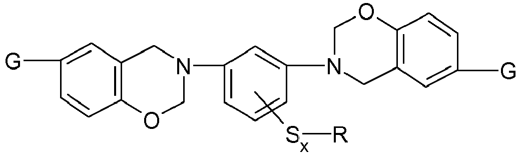

Dans un tel cas, on comprendra que la polybenzoxazine issue du composé de l'invention a donc quant à elle pour caractéristique essentielle de comporter au moins des unités structurelles récurrentes comportent (au moins) un motif répondant aux formule (I-a) (avant ouverture des cycles oxazine) ou formule (11-a) (après ouverture des cycles) ci-dessous :

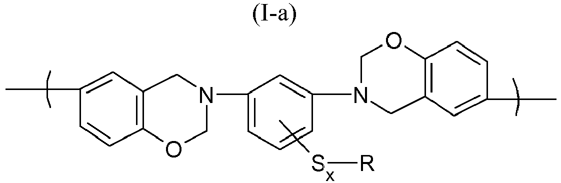

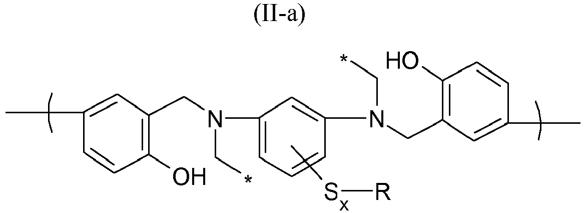

Encore plus préférentiellement, « x » est égal à 1 et R représente un méthyle.

Ainsi, à titre d'exemples de diamines aromatiques sulfurées convenant pour la synthèse d'une benzoxazine de formule (A) ou (A-0) selon l'invention dans laquelle, selon un mode de réalisation particulièrement préférentiel, « x » est égal à 1 et R représente un méthyle, on citera en particulier les composés 3,5-bis(méthylthio)-2,4-toluènediamine, 3,5-bis(méthylthio)-2,6-toluènediamine et leurs mélanges, répondant respectivement aux formules (a) et (b) ci-dessous :

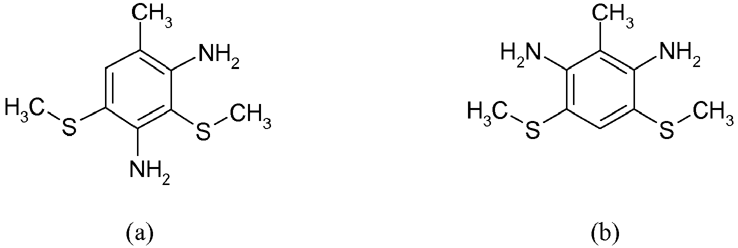

En d'autres termes, selon un mode de réalisation particulièrement préférentiel, si la benzoxazine de formule (A-0) est issue d'au moins un des deux isomères ci-dessus ou de leurs mélanges, alors la polybenzoxazine issue du composé de l'invention comporte des unités récurrentes comportent au moins un motif répondant aux formules (I-a-1) ou (I-b-1) (avant ouverture des cycles oxazine), (II-a-1) ou (11-b-1) (après ouverture des cycles) ci-dessous :

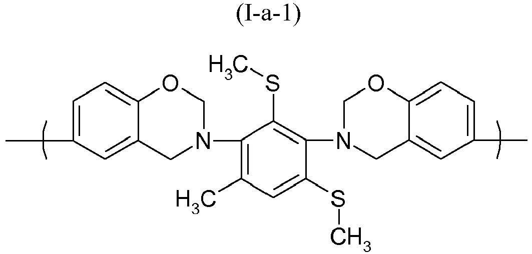

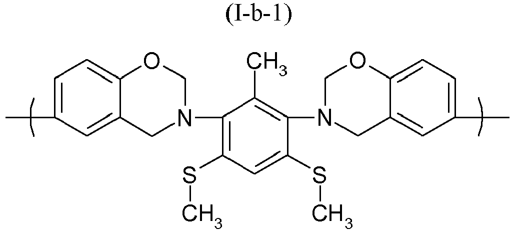

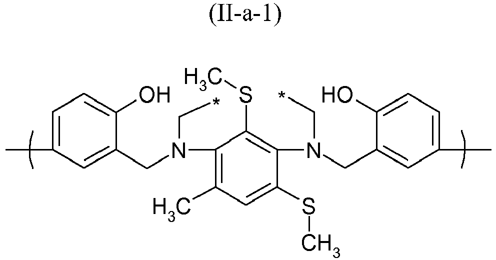

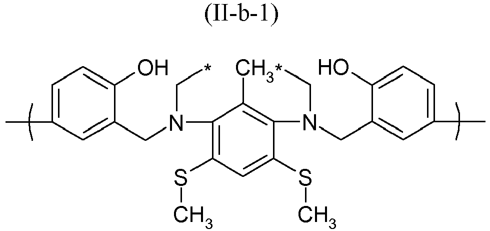

Selon un mode de réalisation préférentiel de l'invention, les (au moins deux) radicaux G, identiques ou différents, représentent un halogène tel que brome, chlore, fluor ou iode.

La figure 3 annexée donne un schéma de synthèse général, sous apport thermique et avec élimination d'eau, à partir d'un phénol halogéné porteur d'au moins un (c'est-à-dire un ou plusieurs) halogène (représenté par le symbole « Hal »), p-formaldéhyde et de la diamine aromatique sulfurée spécifique de la figure 2 précédente, d'une benzoxazine halogénée particulière de formule (A-1) (Monomère noté M-1), conforme à l'invention, utilisable pour la synthèse d'une polybenzoxazine. Cet halogène (Hal) est plus préférentiellement le brome ou le chlore, encore plus préférentiellement le brome ; ce dernier étant plus préférentiellement encore situé en position para de l'oxygène de chaque cycle oxazine.

Selon un autre mode de réalisation préférentiel, les (au moins deux) radicaux G, identiques ou différents, représentent un groupement choisi parmi -OR₁, -SR₁, -NR₂R₃ ; R₁, R₂ et R₃, identiques ou différents, représentant un alkyle ayant 1 à 4 atomes de carbone.

Selon un autre mode de réalisation préférentiel, les (au moins deux) radicaux G, identiques ou différents, représentent un groupement hydrocarboné (représenté par le symbole « A ») aliphatique comportant 1 à 8 atomes de carbone, ou cycloaliphatique comportant 3 à 8 atomes de carbone, ou aromatique comportant 6 à 12 atomes de carbone, ce groupement « A » hydrocarboné, éthyléniquement saturé ou insaturé, pouvant comportant optionnellement un (au moins un) hétéroatome choisi parmi O, S, N et P.

La figure 4 annexée donne un schéma de synthèse général, sous apport thermique et avec élimination d'eau, à partir d'un phénol halogéné porteur d'au moins un (c'est-à-dire un ou plusieurs) tel groupement « A », de paraformaldéhyde et de la diamine aromatique sulfurée spécifique des figures 2 et 3 précédentes, d'une benzoxazine particulière selon l'invention, de formule (A-2) (Monomère noté M-2), utilisable pour la synthèse d'une polybenzoxazine.

La figure 5 donne un autre schéma de synthèse possible, à partir d'un phénol halogéné, de paraformaldéhyde et d'un exemple spécifique de diamine aromatique disulfurée, à savoir la 3,5-bis(méthylthio)-2,6-toluènediamine de formule (b) précédente, d'un autre exemple de benzoxazine halogénée particulière conforme à l'invention, de formule (A-3) (Monomère noté M-3), utilisable pour la synthèse d'une polybenzoxazine.

La figure 6 donne un autre schéma de synthèse possible, à partir d'un autre phénol (le symbole « A » a été décrit précédemment), de paraformaldéhyde et de la 3,5-bis(méthylthio)-2,6-toluènediamine de formule (b) précédente, d'un autre exemple de benzoxazine de formule (A-4) (Monomère noté M-4) utilisable pour la synthèse d'une polybenzoxazine.

Selon un mode de réalisation préférentiel, les (au moins deux) groupements « A », identiques ou différents, représentent un groupement hydrocarboné aliphatique, éthyléniquement saturé ou insaturé, comportant 1 à 6, en particulier 1 à 4 atomes de carbone, pouvant comporter optionnellement au moins un (c'est-à-dire un ou plusieurs) hétéroatome choisi parmi O, S, N et P.

Ainsi, selon un autre mode de réalisation particulier et préférentiel, la benzoxazine disulfurée de l'invention répond au moins en partie à l'une des deux formules (A-5) et (A-5bis) (Monomères respectivement notés M-5 et M-5bis) ci-dessous :

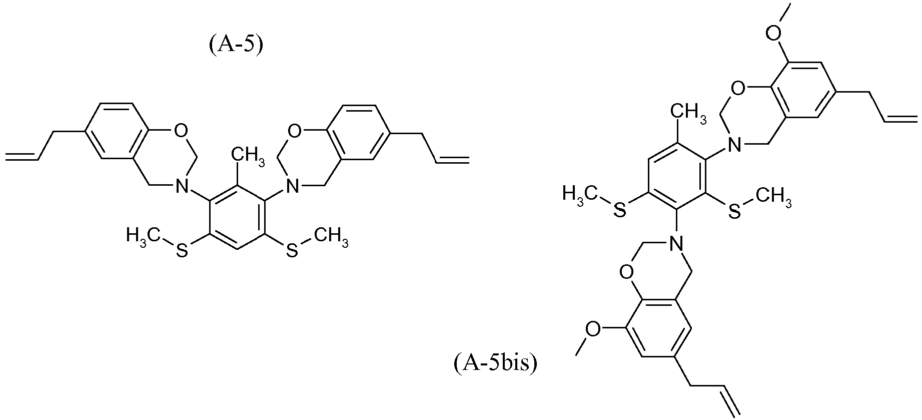

La figure 7 est un cas particulier de la figure 6, qui décrit un autre schéma de synthèse, à partir cette fois d'un exemple particulier d'un phénol (Composé 1) répondant à une telle définition préférentielle (ici, phénol porteur d'une insaturation éthylénique et d'un groupe méthoxyle), de paraformaldéhyde (Composé 2)et de l'exemple particulier de diamine aromatique disulfurée précédente (Composé 3), d'un autre exemple de benzoxazine conforme à l'invention, de formule (A-5) (Monomère noté M-5), utilisable notamment pour la synthèse d'une polybenzoxazine sulfurée.

L'homme du métier saura bien entendu adapter largement la formule spécifique (A) ou (A-0) de la benzoxazine de l'invention servant de monomère de départ pour la synthèse de polybenzoxazines, en faisant varier notamment les formules du phénol (porteur du radical ou des radicaux G) et de la diamine sulfurée (porteur du ou des groupements de formule -Sₓ-R).

A titre d'exemples de diamines aromatiques sulfurées préférentielles, ont déjà été cités notamment les composés 3,5-bis(méthylthio)-2,4-toluènediamine, 3,5-bis(méthylthio)-2,6-toluènediamine et leurs mélanges.

A titre d'exemples de composés phénols (ici, par exemple méthoxyphénols) porteurs de groupements « A » du type hydrocarbonés aliphatiques, éthyléniquement saturés ou insaturés, comportant 1 à 6, en particulier 1 à 4 atomes de carbone, pouvant comporter optionnellement au moins un (c'est-à-dire un ou plusieurs) hétéroatome choisi parmi O, S, N et P, peuvent être cités par exemple les composés suivants :

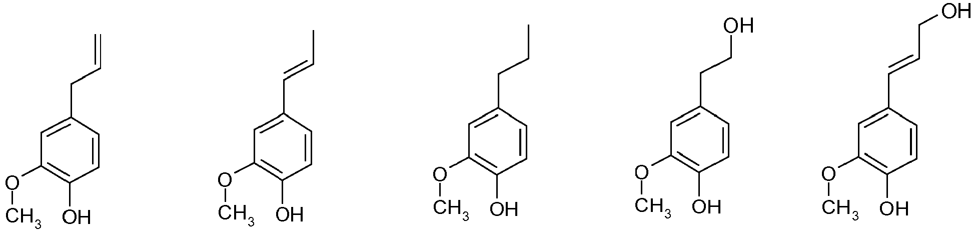

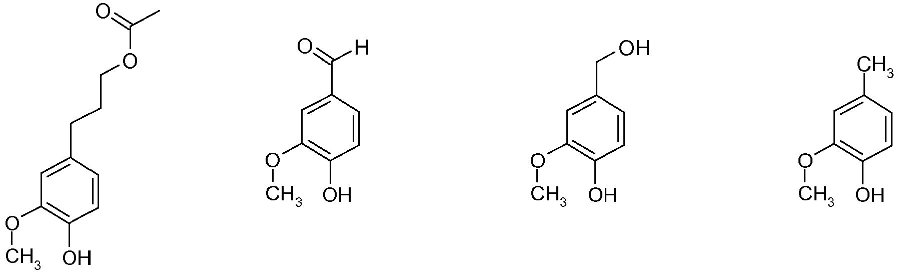

La benzoxazine conforme à l'invention de formule (A) précédemment décrite est particulièrement destinée (à titre de Monomère M) à la synthèse d'une polybenzoxazine par polycondensation, en particulier par polycondensation avec au moins un composé diol ou thiol aromatique à titre de deuxième monomère (« Monomère N »).

Ce composé diol ou thiol aromatique répond plus préférentiellement à la formule (B):

(B) HX₁-Ar₁-Z-Ar₂-X₂H

dans laquelle :
- X₁ et X₂, identiques ou différents, représentent O ou S ;
- Ar₁ et Ar₂, identiques ou différents, représentent un groupe aromatique, de préférence phénylène ;
- Z représente O ou (S)ₙ, le symbole « n » représentant un nombre entier égal ou supérieur à 1.

Ainsi, selon un mode de réalisation particulièrement préférentiel, la polybenzoxazine issue de la benzoxazine sulfurée de l'invention se caractérise par des unités récurrentes comportant au moins un motif répondant aux formules particulières (1-1) (avant ouverture des cycles oxazine) ou (11-1) (après ouverture des cycles) :

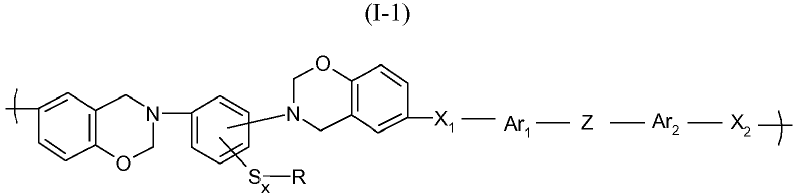

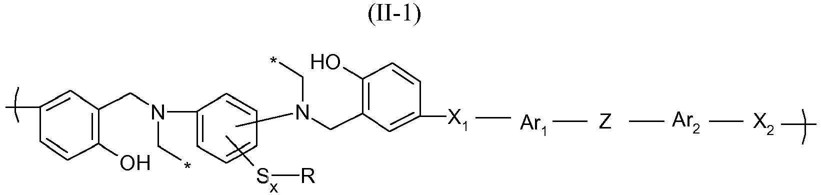

Ici encore, on note bien entendu que, dans les formules ci-dessus, les deux atomes d'azote des cycles oxazine sont, l'un par rapport à l'autre, dans une position quelconque (c'est-à-dire ortho, méta ou para) sur le noyau benzénique central qui les sépare.

Toutefois, plus préférentiellement encore, dans les formules (1-1) et (11-1) ci-dessus, ces deux atomes d'azote sont en position méta l'un par rapport à l'autre sur le noyau benzénique central qui les sépare. En d'autres termes, la polybenzoxazine issue de la benzoxazine de l'invention comporte alors des unités structurelles récurrentes comportant (au moins) un motif répondant aux formule (I-1bis) (avant ouverture des cycles oxazine) ou formule (II-1bis) (après ouverture des cycles) ci-après :

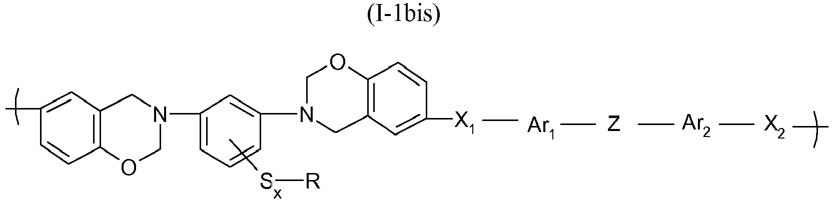

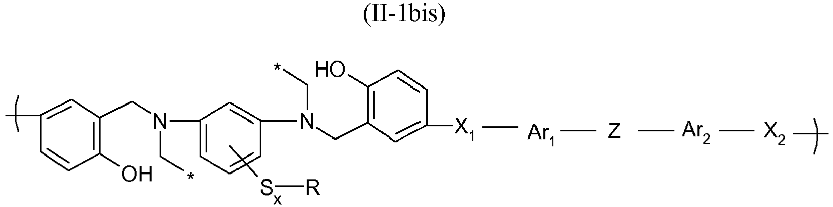

Dans les formules (1-1), (11-1), (I-1bis), (II-1bis) ci-dessus, un ou plusieurs atomes d'hydrogène d'au moins un ou de chaque noyau aromatique Ar₁ et Ar₂ pourraient être substitués par divers substituants, identiques ou différents, par exemple par des groupes fonctionnels susceptibles de favoriser l'adhésion du polymère au métal et/ou au caoutchouc.

La figure 8 décrit un schéma de synthèse général d'une polybenzoxazine sulfurée (Polymère noté P-1) de formule (I-1bis) ci-dessus, conformément au procédé de l'invention, par polycondensation de la benzoxazine halogénée selon l'invention de formule (A-6) (Monomère M-6) précédente avec un autre monomère de formule générique (B) (Monomère noté « N ») du type diol ou thiol aromatique ; ainsi que cet exemple de polybenzoxazine sulfurée (Polymère noté ici P-1' de formule II-1bis) une fois ses cycles oxazine ouverts après traitement thermique du Polymère P-1.

Dans les formules générales (1-1), (11-1), (I-1bis) ou (II-1bis) ci-dessus, on a préférentiellement au moins une des caractéristiques suivantes qui est vérifiée :
- Ar₁ et Ar₂ représentent chacun un groupe phénylène non substitué ;
- X₁ et X₂ représentent chacun soit un atome de soufre, soit un atome d'oxygène ;
- Z représente O ou S (soit « n » égal à 1), plus préférentiellement S.

Plus préférentiellement, c'est l'ensemble des caractéristiques préférentielles ci-dessus qui est vérifié simultanément.

A titre d'exemples préférentiels, le composé de formule (B) précédente répond à au moins une des formules particulières (B-1), (B-2) ou (B-3) ci-dessous :

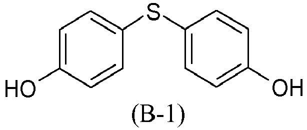

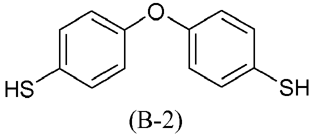

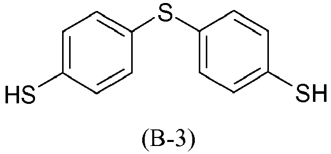

Selon un autre mode de réalisation particulier et préférentiel, le polymère polybenzoxazine peut être obtenu par homopolymérisation d'une benzoxazine de formule (A) ou (A-0) telle que décrite supra.

Ainsi, la figure 9 illustre un schéma de synthèse d'une autre polybenzoxazine (Polymère P-2' de formule II-2), avec ses cycles oxazine ouverts, obtenue cette fois par simple homopolymérisation de la benzoxazine halogénée particulière selon l'invention de formule (A-5) (Monomère M-5) précédente.

Selon un autre mode de réalisation particulier et préférentiel, la benzoxazine de l'invention est une benzoxazine borée et disulfurée répondant au moins en partie à l'une des deux formules (A-7) et (A-7bis) (Monomères respectivement notés M-7 et M-7bis) ci-dessous :

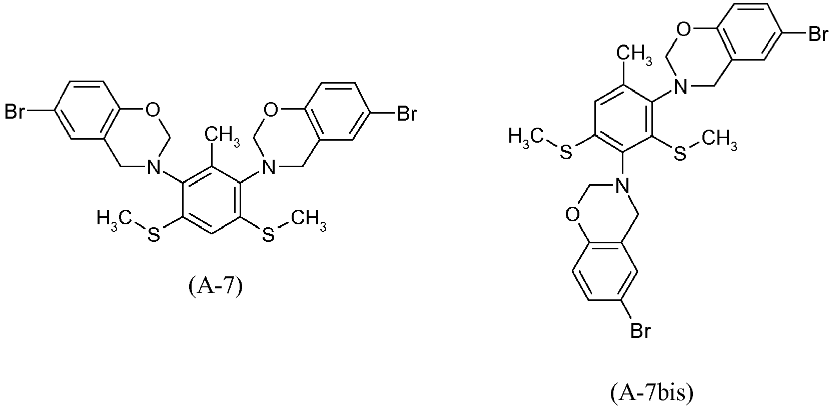

La figure 10 donne précisément un exemple de synthèse, à partir de phénol bromé (composé 4), p-formaldéhyde (composé 2) et de la 3,5-bis(méthylthio)-2,6-toluènediamine (composé 3), de cette benzoxazine bromée disulfurée de formule (A-7) (Monomère noté M-7) utilisable pour la synthèse de polybenzoxazines (Polymère P-3 et P-3' de la figure 11) conformément au procédé de l'invention.

Dans cet exemple de la figure 10, comme pour les figures 7 à 9 précédentes, on note en particulier que, selon un mode de réalisation particulièrement préférentiel de l'invention déjà indiqué, chaque noyau benzénique des deux cycles oxazine de la benzoxazine de formule (A) est porteur d'un et un seul halogène (Hal), plus préférentiellement du brome, situé en position para de l'oxygène du cycle oxazine.

Enfin, la figure 11 décrit la synthèse d'une polybenzoxazine sulfurée (Polymère P-3) à partir de la benzoxazine halogénée particulière de formule (A-7) (Monomère M-7) ci-dessus et d'un autre monomère particulier de formule (B-1) (Monomère N-1) du type diol aromatique soufré (porteur d'une fonction thioéther), ainsi que la structure de ce polymère une fois ses cycles oxazine ouverts (Polymère noté P-3').

Les synthèses des figures 7, 10 et 11 seront décrites plus en détail dans les exemples de réalisation qui suivent.

Typiquement, la polybenzoxazine issue du composé benzoxazine de l'invention peut comporter de dix à plusieurs centaines, préférentiellement de 50 à 300 unités structurelles à motifs de formule (1) et/ou (II), en particulier d'unités structurelles telles que représentées à titre d'exemples aux figures 8, 9 et 11.

Cette polybenzoxazine issue de la benzoxazine de l'invention est avantageusement utilisable, à titre de primaire d'adhésion ou comme couche adhésive unique, pour revêtir un substrat en métal, tout au moins un substrat dont au moins la surface est au moins en partie métallique, et faire adhérer ce dernier à du caoutchouc. Elle est tout particulièrement utilisable sur tout type de renfort métallique, tel que par exemple un fil, un film ou un câble en acier, notamment en acier au carbone, destiné en particulier à renforcer une matrice de caoutchouc insaturé tel que du caoutchouc naturel. Pour faire adhérer le caoutchouc à la couche de polybenzoxazine, on pourra aussi utiliser tout système adhésif connu, par exemple une colle textile conventionnelle du type "RFL" (résorcinol-formaldéhyde-latex). L'homme du métier comprendra aisément que la connexion entre le substrat métallique pourvu de sa couche de polybenzoxazine et la couche de caoutchouc avec laquelle il est au contact, sera assurée définitivement lors de la cuisson (réticulation) finale de l'article en caoutchouc concerné.

### 5. EXEMPLES DE REALISATION DE L'INVENTION

Dans la présente demande, sauf indication expresse différente, tous les pourcentages (%) indiqués sont des % en masse.

Les essais qui suivent décrivent tout d'abord la synthèse de deux exemples de composés benzoxazine (Monomères M-5 et M-7) conformes à l'invention, puis celle d'une polybenzoxazine (Polymère P-3) à partie du Monomère M-7. Enfin, des tests d'adhésion sont conduits pour illustrer l'excellente performance adhésive des polybenzoxazines issues des composés de l'invention.

### 5.1. Synthèse d'une benzoxazine sulfurée selon l'invention (Monomère M-5)

On dispose pour cette synthèse d'un ballon rond à trois cols de 100 ml, équipé d'un thermomètre, d'une entrée d'azote, d'un agitateur magnétique et d'un réfrigérant.

La synthèse est réalisée selon le mode opératoire schématisé à la figure 7, comme expliqué en détail ci-après, à partir de trois composés : un phénol spécifique porteur d'une insaturation éthylénique et d'un groupe méthoxyle (composé 1 ; « Eugenol », produit Aldrich E51791), paraformaldéhyde (composé 2 ; produit Aldrich 158127), et une diamine aromatique disulfurée (composé 3 ; 3,5-bis(méthylthio)2,6-toluènediamine), en présence de deux solvants (toluène et éthanol anhydres).

Le composé 3 a été isolé, par chromatographie sur gel silice, à partir du produit « Ethacure 300 » (fournisseur Albemarle, Belgique), disponible sous forme d'un liquide relativement visqueux, de couleur brunâtre ; il est constitué à environ 96% d'un mélange d'isomères 3,5-bis(méthylthio)-2,4-toluènediamine et de 3,5-bis(méthylthio)2,6-toluènediamine (rapport pondéral d'environ 4/1 d'après analyse chromatographique).

On verse dans le ballon le composé 1 (2 eq ; 4,93 g soit 30 mmol) puis de l'éthanol (51 ml). La présence d'éthanol est ici importante, empêchant la formation de produit intermédiaire du type triazine, instable. Sous agitation, on introduit ensuite le composé 3 (1 eq ; 3,215 g soit 15 mmol), le composé 2 (4 eq ; 1,80 g soit 60 mmol) et enfin le toluène (102 ml). Le milieu réactionnel est chauffé (environ 75°C) à reflux pendant 4 h puis, après distillation de l'éthanol, à environ 100°C pendant 16 h ; enfin, les solvants et résidus volatils sont distillés à 40°C (sous vide de 20 mbar) pour évaporation. Le produit final est ensuite lavé (100 ml méthanol) et séché.

Cette poudre est placée dans du méthanol (50 ml pour 4 g de poudre) et on chauffe à reflux (65°C) pendant 30 min. Puis on laisse la solution refroidir à température ambiante (environ 20°C) pour cristallisation du monomère. Le produit solide obtenu est isolé par filtration (filtre Büchner). On obtient ainsi, après un séchage à l'étuve sous vide à 50°C durant toute une nuit, une poudre dont le spectre RMN ¹H (500 MHz) (solvant THF-d8) a confirmé la structure chimique du Monomère M-5 ainsi synthétisé, avec les résultats suivants:
*2.07 (s, 3H), 2.38 (s, 6H), 3.25 (t, 4H), 3.71 (s, 6H), 3.94-4.01 (t, 2H), 4.58-4.64 (dd, 2H), 4.81-4.86 (dd, 2H), 4.96-5.05 (m, 6H), 5.85-5.97 (m, 2H), 6.37-6.40 (d, 2H), 6.55 (s, 2H), 6.72 (s, 1H).*

### 5.2. Synthèse d'une autre benzoxazine sulfurée selon l'invention (Monomère M-7)

Comme précédemment, on dispose pour cette synthèse d'un ballon rond à trois cols de 100 ml, équipé d'un thermomètre, d'une entrée d'azote, d'un agitateur magnétique et d'un réfrigérant.

La synthèse est réalisée selon le mode opératoire schématisé à la figure 10, comme expliqué en détail ci-après, à partir de trois composés : un phénol halogéné (composé 4 ; 4-bromophénol ; produit Aldrich B75808), une p-formaldéhyde (composé 2 ; produit Aldrich 158127), et une diamine aromatique disulfurée (composé 3 ; 3,5-bis(méthylthio)-2,6-toluènediamine, isolée comme précédemment à partir du produit « Ethacure 300 ») en présence de deux solvants (toluène et éthanol anhydres).

On verse dans le ballon le composé 4 (2 eq ; 2,6 g soit 15 mmol) puis de l'éthanol (23 ml). La présence d'éthanol est ici importante, empêchant la formation de produit intermédiaire du type triazine, instable. Sous agitation, on introduit ensuite le composé 3 (1 eq ; 1,6 g soit 7,5 mmol), le composé 2 (4 eq ; 0,90 g soit 30 mmol) et enfin le toluène (46 ml). Le milieu réactionnel est chauffé (environ 75°C) à reflux pendant 16 h, puis les solvants et résidus volatils sont distillés à 40°C sous vide (20 mbar) pour évaporation.

Le produit final est ensuite placé dans du méthanol (50 ml pour 4,5 g de produit) et on chauffe à reflux (65°C) pendant 30 min. Puis on laisse la solution refroidir à température ambiante (environ 20°C) pour cristallisation du monomère. Le produit solide obtenu est isolé par filtration (filtre Büchner). On obtient ainsi une poudre jaune, après un séchage à l'étuve sous vide à 50°C, durant toute une nuit (rendement de réaction égal à environ 82%).

Les spectres RMN ¹H (500 MHz) du Monomère M-7 ainsi synthétisé, dissous dans un solvant deutéré, ont confirmé sa structure chimique, avec les résultats suivants :
- dans THF-*d8* :
   *2.06 (s, 3H), 2.39 (s, 6H), 4.03-4.14 (t, 2H), 4.59-4.63 (d, 2H), 4.87-4.91 (dd, 2H), 5.01-5.05 (dd, 2H), 6.71-6.74 (d, 2H), 6.81 (s, 1H), 7.15-7.21 (m, 4H)* ;
- dans CD₂Cl₂ :
   *2.06 (s, 3H), 2.39(s, 6H), 4.03-4.14 (t, 2H), 4.53-4.58 (dd, 2H), 4.92-4.95 (dd, 2H), 5.00-5.04 (dd, 2H), 6.68 (s, 1H), 6.74-6.77(d, 2H), 7.14-7.15 (d, 2H), 7.21-7.24 (dd, 2H).*

### 5.3. Synthèse d'une polybenzoxazine polysulfurée (Polymère P-3)

Cette synthèse est réalisée selon le mode opératoire schématisé à la figure 11, comme décrit en détail ci-après, à partir de deux monomères : la benzoxazine de l'invention obtenue à l'étape précédente (Monomère M-7) et le diol aromatique soufré de formule (B-1) (4,4'-thiodiphénol ; Monomère N-1) ; ceci en présence de carbonate de sodium (Na₂CO₃ ; produit Sigma Aldrich 13418), de solvants (anhydres) DMA (N,N-diméthylacétamide ; produit Sigma Aldrich 38839) et toluène (produit Acros Organics N°364411000). Les deux monomères (M-7 et N-1) sont préalablement séchés sous vide (10 mbar) à 60°C toute la nuit, de même pour le carbonate de sodium mais à une température de 150°C.

La synthèse est conduite dans un ballon rond à quatre cols de 100 ml, équipé d'une entrée d'azote, d'un thermomètre, d'un agitateur magnétique et d'un séparateur « Dean Stark » surmonté d'un réfrigérant et d'un pont de distillation (pourvu d'une calotte chauffante). L'appareillage est séché sous vide en utilisant un pistolet à air chaud jusqu'à ce que le thermomètre atteigne une température d'au moins 100°C dans le ballon de réaction. On laisse l'ensemble refroidir à température ambiante (20°C), puis l'appareillage est mis sous courant d'azote pendant toute la synthèse.

On introduit alors dans le ballon tout d'abord le Monomère M-7 (1 eq ; 1,5 g soit 2,79 mmol) de formule (A-7), puis le Monomère N-1 de formule (B-1) (1 eq ; 0,61 g soit 2,79 mmol). On ajoute ensuite 20 ml de DMA (solvant des deux monomères), puis à titre de base Na₂CO₃ (3 eq ; 0,89 g soit 8,36 mol) en suspension dans 4 ml de toluène. Le tout est purgé sous N₂ pendant 5 min, puis on chauffe le milieu réactionnel à 105°C. Une fois cette température atteinte (température de calotte chauffante d'environ 115°C), le pont de distillation de l'appareillage Dean Stark est chauffé à 110°C (avec la calotte chauffante) afin de faciliter la distillation azéotropique (distillation eau/toluène) conduite pendant environ 90 min. Puis on augmente progressivement la température du milieu réactionnel, par pallier de 10°C toutes les 30 min, jusqu'à atteindre 130°C. On laisse à cette température durant 17 h, puis on laisse refroidir à température ambiante (20°C). Le mélange réactionnel est ensuite distillé à 90°C (vide 3 mbar) pour élimination des solvants et résidus volatils, puis le précipité solide ainsi obtenu est lavé avec 250 ml d'eau distillée ; au cours de ce lavage, pour extraire le carbonate, on ajoute de l'acide (HCl 1 % aqueux) goutte à goutte jusqu'à pH neutre. Le précipité est une nouvelle fois lavé avec 100 ml d'eau distillée, séché sous vide à 80°C pendant toute une nuit (environ 12 h) ; le polymère P-3 de la figure 11 a été ainsi obtenu, comme attesté par l'analyse RMN ¹H (500 MHz).

Le Polymère P-3 de la Fig. 11 a été ainsi obtenu, comme attesté par l'analyse RMN ¹H (500 MHz) dans le solvant THF-*d8*, qui a donné les résultats suivants :
*1.92 (s, 3H), 2.26 (s, 6H), 3.74-3.81 (m, 4H), 4.01-4.03 (t, 2H), 4.75-5.01 (m, 2H), 6-15-6.75 (m, 4H), 6.90-7.45 (br, 11H).*

Ce Polymère P-3, sous forme d'une poudre de couleur jaune clair, a été également analysé par DSC (*Differential Scanning Calorimetry*) entre -80°C et +350°C selon une rampe de 10°C/min (appareil DSC "822-2" de Mettler Toledo ; atmosphère azote). L'analyse a montré au premier passage (entre -80°C et +350°C) une transition vitreuse apparente (Tg) à 163°C suivie par une exothermie (correspondant à l'ouverture des cycles oxazine, et à la réticulation du polymère) au-delà de 200°C, avec deux maxima à environ 270°C et 299°C. Au cours des deuxième et troisième passages de DSC conduits entre -80°C et +350°C, aucune transition vitreuse apparente n'a été visible.

### 5.4. Test d'adhésion dans un composite métal / caoutchouc

Une partie (325 mg) du Polymère P-3 précédemment préparé a été dissoute dans 8 ml de DMPU (1,3-diméthyl-3,4,5,6-tetrahydro-2(1H)-pyrimidinone ; produit Sigma Aldrich 41661) avec 10% en poids d'accélérateur « DY 9577 ES » (produit Huntsman), ceci pour former une solution dont une fraction (0,6 ml) a été ensuite déposée de manière uniforme sur un ruban (film) en laiton de dimensions 10 cm x 2,5 cm et d'épaisseur 0,5 mm ; l'ensemble a été placé à l'étuve à 175°C (ventilation à l'air) durant 5 min, puis 5 min supplémentaires à 230°C sous vide afin d'une part d'éliminer toute trace de solvant et d'autre part d'ouvrir au moins en partie (c'est-à-dire totalement ou partiellement) les cycles oxazine du polymère, cette dernière étape s'accompagnant d'un changement prononcé de couleur du polymère, passant au orange foncé.

Après refroidissement à température ambiante, le ruban pourvu en surface de sa fine couche (épaisseur 5 à 10 µm) de polybenzoxazine ainsi formée, a été ensuite soumis à une opération d'encollage conventionnelle en deux temps (encollage deux bains), tout d'abord par immersion dans un premier bain aqueux (environ 94% d'eau) à base de résine époxy (polyglycérol polyglycidyl éther, environ 1%) et de composé isocyanate (bloqué caprolactame, environ 5%), première étape d'encollage suivie d'un séchage (2 min à 100°C) puis d'un traitement thermique (5 min à 200°C). Puis le ruban ainsi traité a été immergé dans un second bain aqueux de colle RFL (environ 81% en poids d'eau) à base de résorcinol (environ 2%), formol (environ 1%) et d'un latex de caoutchouc (environ 16% de caoutchoucs NR, SBR et VP-SBR) ; il a été enfin séché au four pendant 2 min à 130°C, puis traité thermiquement 5 min à 200°C.

Le ruban en laiton ainsi revêtu du film de polybenzoxazine puis encollé, a été ensuite placé entre deux couches de composition de caoutchouc conventionnelle pour armature de ceinture de bandage pour véhicule tourisme, composition à base de caoutchouc naturel, de noir de carbone et silice à titre de charge, et d'un système de vulcanisation (soufre et accélérateur sulfénamide) ; cette composition étant dépourvue de sel de cobalt. Puis l'éprouvette de composite métal / caoutchouc ainsi préparée a été placée sous presse et le tout cuit (vulcanisé) à 150°C pendant 30 min sous une pression de 20 bar.

Après vulcanisation du caoutchouc, on a obtenu un excellent collage entre la matrice de caoutchouc et le ruban métallique malgré l'absence de sel de cobalt dans la matrice de caoutchouc : lors de tests de pelage (à 20°C), on a constaté en effet que la rupture se produisait systématiquement dans la matrice de caoutchouc elle-même et non à l'interphase entre métal et caoutchouc. D'autres essais de collage ont été conduits sur un ruban en acier clair (non revêtu) ; ils ont révélé eux aussi une excellente adhésion au caoutchouc (rupture systématique dans la matrice de caoutchouc).

En conclusion, la benzoxazine selon l'invention permet la synthèse de polymères offrant aux renforts métalliques l'avantage majeur de pouvoir ensuite être collés à des matrices de caoutchouc en utilisant de simples colles textiles telles que des colles RFL, ou encore directement (c'est-à-dire sans emploi de telles colles) à ces matrices de caoutchouc, par exemple lorsque ces dernières contiennent des élastomères insaturés fonctionnalisés appropriés tels que des élastomères époxydés. Ainsi, peuvent être utilisés des renforts métalliques revêtus ou non de couches métalliques adhésives telles que du laiton, ainsi que des matrices de caoutchouc environnantes dépourvues de sels métalliques, en particulier de sels de cobalt.

En outre, ceci constituant un avantage notable comparativement aux autres polymères connus décrits en introduction du présent mémoire, les polybenzoxazines issues des benzoxazines de l'invention ont la capacité remarquable, à haute température, d'ouvrir leurs cycles oxazine et de conduire ainsi à une structure de résine polyphénolique thermodurcissable. Ceci leur confère une meilleure stabilité thermique. Leur microstructure spécifique permet enfin, très avantageusement, d'ajuster la flexibilité de la molécule selon les applications particulières visées.

## Revendications

1. Composé benzoxazine sulfurée répondant à la formule : 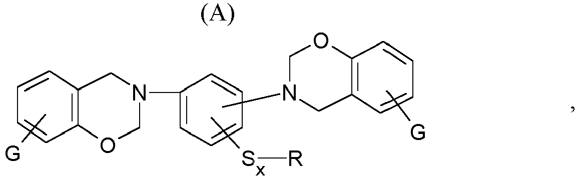 dans laquelle :
- chaque noyau benzénique des deux cycles oxazine est porteur d'au moins un radical noté « G » ;
- les deux cycles oxazine sont reliés entre eux par un groupe aromatique central dont le cycle benzénique est porteur de un, deux, trois ou quatre groupements de formule -Sₓ-R dans laquelle « x » est un entier de 1 à 8 et R représente l'hydrogène ou un groupe hydrocarboné comportant 1 à 10 atomes de carbone et optionnellement un hétéroatome choisi parmi O, S, N et P ;
- les au moins deux radicaux G, identiques ou différents, sont choisis dans le groupe constitué par :
- les halogènes ;
- les groupements -OR₁, -SR₁, -NR₂R₃ ; R₁, R₂ et R₃, identiques ou différents, représentant un alkyle ayant 1 à 4 atomes de carbone ; et
- les groupements hydrocarbonés aliphatiques comportant 1 à 8 atomes de carbone, ou cycloaliphatiques comportant 3 à 8 atomes de carbone, ou aromatiques comportant 6 à 12 atomes de carbone, ces groupements hydrocarbonés comportant en outre optionnellement au moins un hétéroatome choisi parmi O, S, N et P ; et dans laquelle le noyau benzénique pourrait être porteur d'éventuels autres substituants tels qu'un groupe méthyle ou éthyle.

2. Composé selon la revendication 1, les deux atomes d'azote des cycles oxazine étant, l'un par rapport à l'autre, en position méta sur le cycle benzénique qui les sépare.

3. Composé selon la revendication 1 ou 2, le cycle benzénique central étant porteur de deux groupements de formule -Sₓ-R.

4. Composé selon la revendication 3, les deux groupements de formule -Sₓ-R étant en position méta l'un par rapport à l'autre sur le cycle benzénique central.

5. Composé selon l'une quelconque des revendications 1 à 4, chaque noyau benzénique des deux cycles oxazine étant porteur d'un et un seul radical G.

6. Composé selon la revendication 5, le radical G porté par chaque noyau benzénique des deux cycles oxazine étant situé en position para de l'oxygène du cycle oxazine.

7. Composé selon l'une quelconque des revendications 1 à 6, les radicaux G, identiques ou différents, représentant un halogène, de préférence l'halogène étant le brome ou le chlore, de préférence le brome.

8. Composé selon les revendications 6 et 7, répondant à l'une des formules (A-7) ou (A-7bis) suivantes :

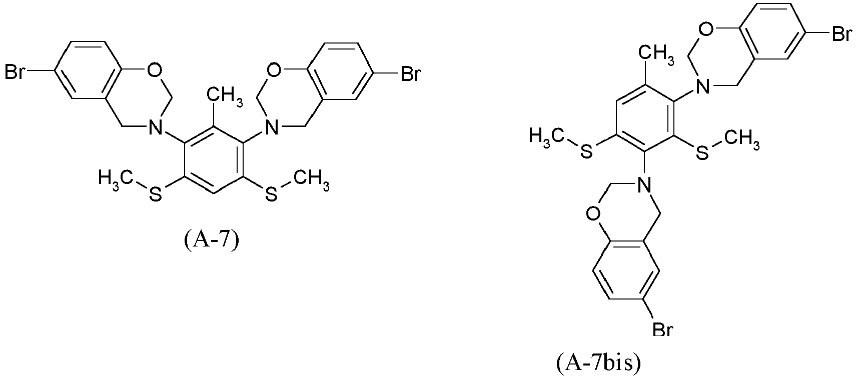

9. Composé selon l'une quelconque des revendications 1 à 6, les radicaux G, identiques ou différents, représentant un groupement hydrocarboné aliphatique comportant 1 à 8 atomes de carbone, ou cycloaliphatique comportant 3 à 8 atomes de carbone, ou aromatique comportant 6 à 12 atomes de carbone, ce groupement hydrocarboné, éthyléniquement saturé ou insaturé, pouvant comportant optionnellement un hétéroatome choisi parmi O, S, N et P.

10. Composé selon la revendication 9, les radicaux G, identiques ou différents, représentant un groupement hydrocarboné aliphatique, éthyléniquement saturé ou insaturé, comportant 1 à 6, de préférence 1 à 4 atomes de carbone.

11. Composé selon les revendications 6 et 10 répondant à l'une des formules (A-5) ou (A-5bis) suivantes :

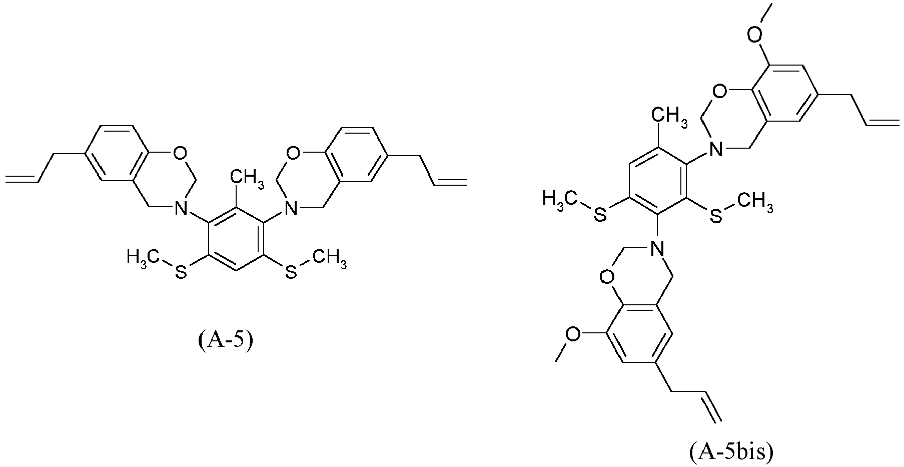

12. Polybenzoxazine issue d'au moins un composé selon l'une quelconque des revendications 1 à 11.

13. Procédé de synthèse d'une polybenzoxazine, par polycondensation d'un composé selon l'une quelconque des revendications 1 à 11.

14. Procédé selon la revendication 13, par polycondensation, à titre de premier monomère, d'un composé selon l'une quelconque des revendications 1 à 11, avec au moins, à titre de deuxième monomère, un composé diol ou thiol aromatique.

15. Procédé selon la revendication 14, le composé diol ou thiol aromatique répondant à la formule (B):
(B) HX₁-Ar₁-Z-Ar₂-X₂H
dans laquelle :
- X₁ et X₂, identiques ou différents, représentent O ou S ;
- Ar₁ et Ar₂, identiques ou différents, représentent un groupe aromatique, de préférence phénylène ;
- Z représente O ou (S)ₙ, le symbole « n » représentant un nombre entier égal ou supérieur à 1.

## Patentansprüche

1. Verbindung schwefelhaltigen Benzoxazins, die der folgenden Formel entspricht: 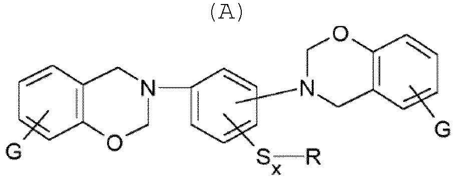 wobei:
- jeder Benzolkern der beiden zyklischen Oxazinbausteine mit mindestens einem Rest versehen ist, der als "G" bezeichnet wird,
- die beiden zyklischen Oxazinbausteine über eine mittige aromatische Gruppe miteinander verbunden sind, deren Benzolring mit einer, zwei, drei oder vier Gruppen der Formel -Sₓ-R versehen ist, in welcher "x" eine ganze Zahl von 1 bis 8 ist und R für Wasserstoff oder eine kohlenwasserstoffartige Gruppe mit 1 bis 10 Kohlenstoffatomen und möglicherweise einem Heteroatom steht, welches aus O, S, N und P ausgewählt ist;
- die mindestens zwei Reste G, welche vollkommen gleichartig oder voneinander verschieden sind, aus der Gesamtheit ausgewählt sind, die aus Folgendem besteht:
- den Halogenen;
- den Gruppen -OR₁, -SR₁, -NR₂N₃; wobei R₁, R₂ und R₃, die vollkommen gleichartig oder voneinander verschieden sind, für ein Alkyl mit 1 bis 4 Kohlenstoffatomen stehen; und
- den kohlenwasserstoffartigen Gruppen aliphatischer Art mit 1 bis 8 Kohlenstoffatomen, oder cycloaliphatischer Art mit 3 bis 8 Kohlenstoffatomen, oder aromatischer Art mit 6 bis 12 Kohlenstoffatomen, wobei diese kohlenwasserstoffartigen Gruppen darüber hinaus möglicherweise mindestens ein Heteroatom aufweisen, welches aus O, S, N und P ausgewählt ist; und wobei der Benzolkern möglicherweise mit weiteren Substituenten, wie etwa einer Methyl- oder Ethylgruppe, versehen sein könnte.

2. Verbindung nach Anspruch 1, wobei sich die beiden Stickstoffatome der zyklischen Oxazinbausteine in meta-Stellung zueinander am Benzolkern befinden, welcher sie trennt.

3. Verbindung nach Anspruch 1 oder 2, wobei der mittige Benzolring mit zwei Gruppen der Formel -Sₓ-R versehen ist.

4. Verbindung nach Anspruch 3, wobei sich die beiden Gruppen mit der Formel -Sₓ-R in meta-Stellung zueinander am mittigen Benzolring befinden.

5. Verbindung nach einem beliebigen der Ansprüche 1 bis 4, wobei jeder Benzolkern der beiden zyklischen Oxazinbausteine mit einem einzigen Rest G versehen ist.

6. Verbindung nach Anspruch 5, wobei der Rest G, mit welchem jeder Benzolkern der beiden zyklischen Oxazinbausteine sich in para-Stellung zum Sauerstoff des zyklischen Oxazinbausteins befindet.

7. Verbindung nach einem beliebigen der Ansprüche 1 bis 6, wobei die Reste G, welche vollkommen gleichartig oder voneinander verschieden sind, für ein Halogen stehen, wobei es sich bei dem Halogen vorzugsweise um Brom oder Chlor, bevorzugt um Brom, handelt.

8. Verbindung nach den Ansprüchen 6 und 7, wobei sie einer der folgenden Formeln (A-7) oder (A7bis) entspricht:

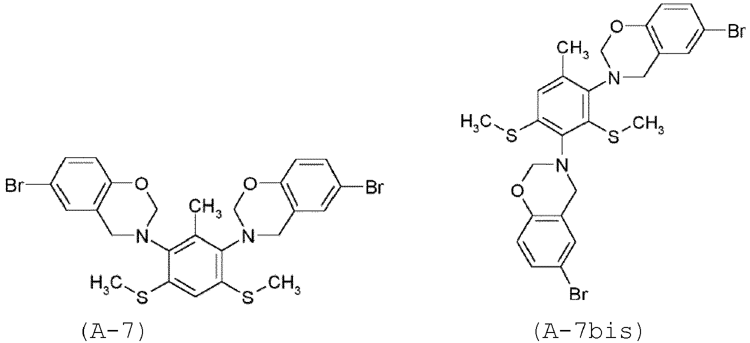

9. Verbindung nach einem beliebigen der Ansprüche 1 bis 6, wobei die Reste G, welche vollkommen gleichartig oder voneinander verschieden sind, für eine kohlenwasserstoffartige Gruppe aliphatischer Art mit 1 bis 8 Kohlenstoffatomen, oder cycloaliphatischer Art mit 3 bis 8 Kohlenstoffatomen, oder aromatischer Art mit 6 bis 12 Kohlenstoffatomen stehen, wobei die kohlenwasserstoffartige Gruppe, welche ethylenisch gesättigt oder ungesättigt ist, möglicherweise ein Heteroatom aufweisen kann, welches aus O, S, N und P ausgewählt ist.

10. Verbindung nach Anspruch 9, wobei die Reste G, welche vollkommen gleichartig oder voneinander verschieden sind, für eine kohlenwasserstoffartige Gruppe aliphatischer Art stehen, die ethylenisch gesättigt oder ungesättigt ist, wobei sie 1 bis 6, vorzugsweise 1 bis 4 Kohlenstoffatome, aufweist.

11. Verbindung nach den Ansprüchen 6 und 10, wobei sie einer der folgenden Formeln (A-5) oder (A-5bis) entspricht:

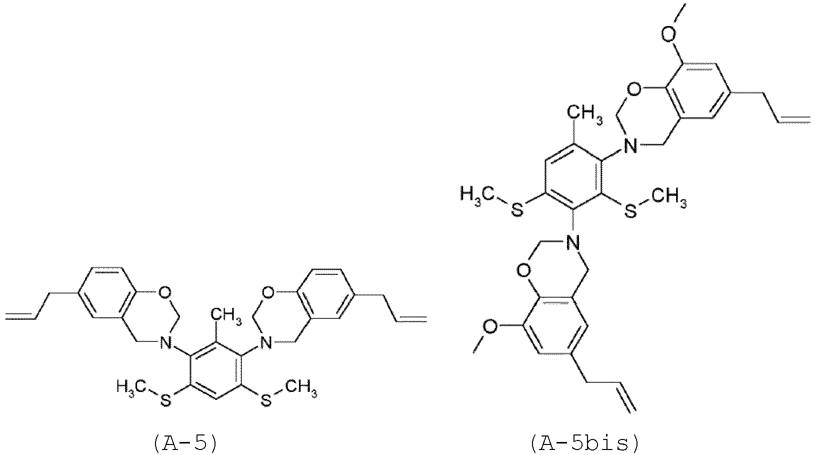

12. Polybenzoxazin, das aus mindestens einer Verbindung nach einem beliebigen der Ansprüche 1 bis 11 hervorgeht.

13. Verfahren zur Synthese eines Polybenzoxazins, mittels Polykondensation einer Verbindung nach einem beliebigen der Ansprüche 1 bis 11.

14. Verfahren nach Anspruch 13, mittels Polykondensation einer Verbindung nach einem beliebigen der Ansprüche 1 bis 11 als erstem Monomer mit mindestens einer aromatischen Diol- oder Thiolverbindung als zweitem Monomer.

15. Verfahren nach Anspruch 14, wobei die aromatische Diol- oder Thiolverbindung der Formel (B) entspricht:
(B) HX₁ --- Ar₁ --- Z --- Ar₂ --- X₂H
wobei:
- X₁ und X₂, die vollkommen gleichartig oder voneinander verschieden sind, für O oder S stehen;
- Ar₁ und Ar₂, die vollkommen gleichartig oder voneinander verschieden sind, für eine aromatische Gruppe, vorzugsweise Phenylen, stehen;
- Z für O oder (S)ₙ steht, wobei das Zeichen "n" für eine ganze Zahl steht, die mindestens 1 beträgt.

## Claims

1. Sulfurized benzoxazine compound corresponding to formula (A): 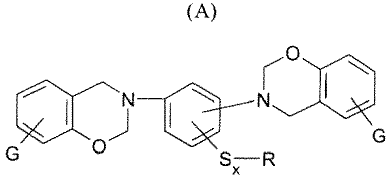 in which:
- each benzene nucleus of the two oxazine rings bears at least one radical denoted as "G";
- the two oxazine rings are connected together via a central aromatic group, the benzene ring of which bears one, two, three or four groups of formula -Sₓ-R in which "x" is an integer from 1 to 8 and R represents hydrogen or a hydrocarbon-based group including 1 to 10 carbon atoms and optionally a heteroatom chosen from O, S, N and P;
- the at least two radicals G, which may be identical or different, are chosen from the group consisting of:
- halogens;
- groups -OR₁, -SR₁, -NR₂R₃; R₁, R₂ and R₃, which may be identical or different, representing an alkyl containing 1 to 4 carbon atoms; and
- aliphatic hydrocarbon-based groups including 1 to 8 carbon atoms, or cycloaliphatic hydrocarbon-based groups including 3 to 8 carbon atoms, or aromatic hydrocarbon-based groups including 6 to 12 carbon atoms, these hydrocarbon-based groups also optionally including at least one heteroatom chosen from O, S, N and P; and in which benzene ring may bear other optional substituents such as methyl or ethyl group.

2. Compound according to Claim 1, the two nitrogen atoms of the oxazine rings being, relative to each other, in the meta-position on the benzene ring which separates them.

3. Compound according to Claim 1 or 2, the central benzene ring bearing two groups of formula -Sₓ-R.

4. Compound according to Claim 3, the two groups of formula -Sₓ-R being in the meta-position relative to each other on the central benzene ring.

5. Compound according to any one of Claims 1 to 8, each benzene nucleus of the two oxazine rings bearing only one radical G.

6. Compound according to Claim 5, the radical G borne by each benzene nucleus of the two oxazine rings being located in the para position relative to the oxygen of the oxazine ring.

7. Compound according to any one of Claims 1 to 6, the radicals G, which may be identical or different, representing a halogen, the halogen being bromine or chlorine, preferably bromine.

8. Compound according to Claims 6 and 7, corresponding to one of the formulae (A-7) or (A-7bis) below:

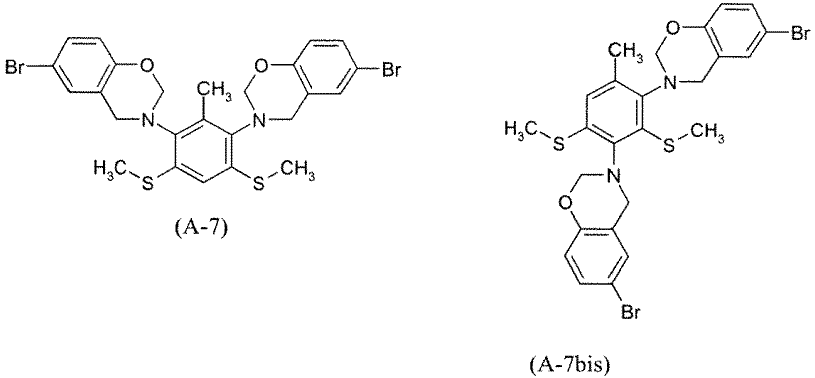

9. Compound according to any one of Claims 1 to 6, the radicals G, which may be identical or different, representing an aliphatic hydrocarbon-based group including 1 to 8 carbon atoms, or a cycloaliphatic hydrocarbon-based group including 3 to 8 carbon atoms, or an aromatic hydrocarbon-based group including 6 to 12 carbon atoms, this saturated or ethylenically unsaturated hydrocarbon-based group being able to optionally include at least one heteroatom chosen from O, S, N and P.

10. Compound according to Claim 9, the radicals G, which may be identical or different, representing a saturated or ethylenically unsaturated aliphatic hydrocarbon-based group including 1 to 6 and preferably 1 to 4 carbon atoms.

11. Compound according to Claims 6 and 10, corresponding to one of the formulae (A-5) or (A-5bis) below:

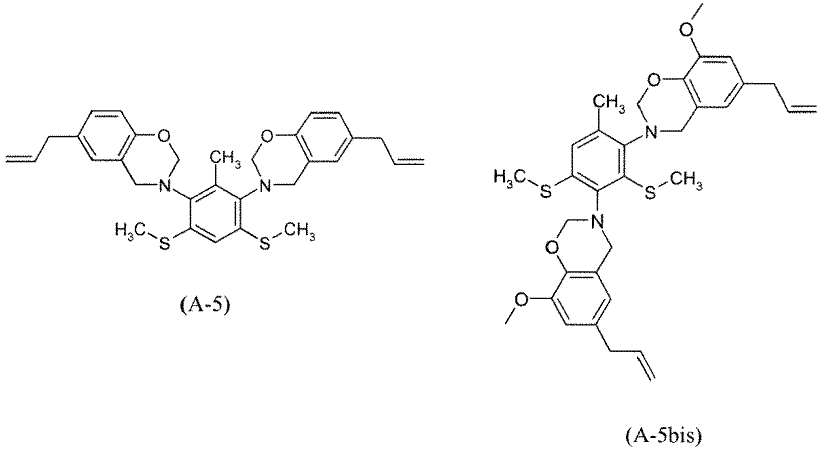

12. Polybenzoxazine derived from at least one compound according to any one of Claims 1 to 11.

13. Process for synthesizing a polybenzoxazine, by polycondensation of a compound according to any one of Claims 1 to 11.

14. Process according to Claim 13, by polycondensation, as first monomer, of a compound according to any one of Claims 1 to 11, with at least, as second monomer, one aromatic diol or thiol compound.

15. Process according to Claim 14, the aromatic diol or thiol compound corresponding to formula (B):
(B) HX₁-Ar₁-Z-Ar₂-X₂H
in which:
- X₁ and X₂, which may be identical or different, represent O or S;
- Ar₁ and Ar₂, which may be identical or different, represent an aromatic group, preferably phenylene;
- Z represents O or (S)ₙ, the symbol "n" representing an integer greater than or equal to 1.
